# EUROPEAN PATENT APPLICATION

(11) **EP 1 087 012 A1**
(43) Date of publication of application: **28.03.2001**
(21) Application number: 00912973.5
(22) Date of filing: 30.03.2000
(51) Int. Cl.: C12N 15/12, C07K 14/47, C07K 16/18

(54) **GROUPS OF GENES EXPRESSED IN HUMAN DENDRITIC CELLS**

(30) Priority: 01.04.1999 JP 9548199
(71) Applicant: Japan Science and Technology Corporation, Kawaguchi-shi, Saitama 332-0012 (JP)
(72) Inventor: HASHIMOTO, Shinichi, Taito-ku, Tokyo 110-0001 (JP); MATSUSHIMA, Kouji, Matsudo-shi, Chiba 271-0092 (JP); SUZUKI, Takuji, Tokyo 143-0023 (JP)
(74) Representative: Gardner, Rebecca
(86) International application number: JP0002019
(87) International publication number: WO0060074

(57) **Abstract**

This invention provides a gene group comprising 100 genes which is highly expressed in a human dendritic cell and provides a gene group which is expressed in a high frequency and is expressed in a low frequency in a human dendritic cell as compared with in human monocyte. The gene group comprising 100 genes which is most abundantly expressed in a human dendritic cell is characterized in that the cDNA of each gene has a nucleotide sequence of each of SEQ ID NO:1 to NO:100 connecting to 5'-CATG-3' which is the nearest nucleotide sequence to a poly A region. This invention also provides a cDNA group of genes constituting each gene group, a novel gene group contained in such a gene group and an oligonucleotide group for specifying such gene and cDNA.

## Description

### Technical Field

The invention of this application relates to a group of genes expressed in dendritic cells derived from human monocyte. More particularly, it relates to an aggregate of genes comprising 100 genes which are highly expressed in human dendritic cells; to an aggregate comprising 100 genes each being highly and lowly expressed in human dendritic cells as compared with in human monocyte; to a cDNA group for each of those gene groups; to novel gene contained in such gene groups; and to an aggregate of oligonucleotides for specifying those genes and cDNAs.

### Background Art

Dendritic cells (DCs) play a very important role in an immune system by, for example, providing CD4⁺-naive T cells with antigen. It has been also reported that DCs directly participate in a T cell tolerance and an immunoglobulin production by B cells. In lymphocytes and nonlymphoid DCs, surface markers and function of each of them are different whereby they have different names. (For example, Langerhans cells in skin; interdigitating DC in lymph node; interstitial DC in heart, lung, kidney and testicle; thymus DC in thymus; etc.)

Although DCs have been believed to belong to a type different from monocyte and macrophage, macrophage and DCs have been reported to be derived from common stem cells. It has been also reported that human DCs are generated from CD34⁺ precursor cells isolated from cord blood and bone marrow in the presence of granulate-macrophage colony stimulating factor (GM-CSF) and tumor necrosis factor α (TNF-α). It is further reported that blood monocyte incubated with GM-CSF and interleukin-4 (IL-4) is differentiated to non-adhesive 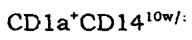 having morphological and functional characteristics of DCs. It is furthermore reported that DCs change to mature DCs expressing CD38 by a stimulation TNF-α, CD40 ligand, LPS or monocyte conditioned medium.

However, it is the present state that, although DCs are known to be the cells differentiated from monocyte, a process for the differentiation has not been clarified at all.

Clarification of a differentiation process of monocyte to DCs is quite important not only for understanding the function of an immune system but also for developing the novel means for diagnosis and therapy of various human diseases in which the dendritic cell is participated. In order to clarify the process of differentiation of monocyte to DCs, it is one of the effective means to specify a gene group which is expressed with a high frequency or a low frequency in each of the cells.

The invention of this application has been achieved under the circumstances as mentioned above and its object is to provide a gene group comprising the preceding 100 genes which are most abundantly expressed among the genes expressed in human dendritic cell.

Another object of this application is to provide a cDNA group comprising each cDNA of the gene group; novel gene contained in the gene group; and an oligonucleotide group for specifying the gene group and the cDNA group.

Still another object of this application is to provide a gene group comprising each of the preceding 100 genes from the highly expressing part and the preceding 100 genes from the lowly expressing part in human dendritic cells derived from human monocyte as compared with in human monocyte. Further object is to provide a cDNA group, a novel gene group and an oligonucleotide group for each of those gene groups.

### Disclosure of Invention

As an invention for solving the above-mentioned problems, this application provides a human dendritic cell-expressed gene group comprising 100 genes at the highest expression frequency among genes expressed in human dendritic cells derived from human monocytes, wherein the cDNAs of the individual genes individually contain nucleotide sequences of SEQ ID N0: 1 to 100 consecutive to the nucleotide sequence 5'-CATG-3' closest to the poly A region; a cDNA group comprising each cDNA of this gene group; a novel gene contained in this gene group; a novel protein encoded by the novel gene; an antibody and antagonist; and an oligonucleotide group comprising each nucleotide sequence of SEQ ID NO: 1 to NO: 100 consecutive to the nucleotide sequence 5'-CATG-3'. Incidentally, in the gene group which most abundantly expresses the human dendritic cell, it is a preferred embodiment that the expression frequency of the gene encoding the cDNA containing the nucleotide sequence of SEQ ID N0: 1 is the highest and the expression frequencies of the remaining 99 genes are in the order of SEQ ID N0: 2 to 100

This application also provides a human dendritid cell-expressed gene group comprising 100 genes expressed more in human dendritic cells derived from human monocytes than in human monocytes, wherein the cDNAs of the individual genes individually contain nucleotide sequence of SEQ ID NO:101 to 200 consecutive to the nucleotide sequence 5'-CATG-3' closest to the poly A region; a cDNA group comprising each cDNA in this gene group; a novel gene group contained in this gene group; a protein encoded by the novel gene; an antibody and an antagonist; and an oligonucleotide group comprising each of the nucleotide sequences of SEQ ID NO:101 to NO:200 consecutive to the nucleotide sequence 5'-CATG-3'. In the gene group where an expression increases in this human dendritic cell, it is a preferred embodiment that the difference between the expression frequency of the gene encoding the cDNA containing the nucleotide sequence of SEQ ID N0: 101 and the expression frequency of this gene in human monocytes is largest, and the expression frequencies of the remaining 99 genes are in the order of SEQ ID N0: 102 to 200.

This application further provides a human dendritid cell-expressed gene group comprising 100 genes expressed less in human dendritic cells derived from human monocytes than in human monocytes, wherein the cDNAs of the individual genes individually contain nucleotide sequence of SEQ ID NO:201 to 300 consecutive to the nucleotide sequence 5'-CATG-3' closest to the poly A region; a cDNA group comprising each cDNA of this gene group; a novel gene group contained in this gene group; a protein encoded by the nobel gene; an antibody and an antagonist; and an oligonucleotide group comprising each of the nucleotide sequences of SEQ ID NO:201 to NO:300 consecutive to the nucleotide sequence 5'-CATG-3'. In the gene group where expression lowers in this human dendritic cell, it is a preferred embodiment that the difference between the expression frequency of the gene encoding the cDNA containing the nucleotide sequence of SEQ ID N0: 201 and the expression frequency of this gene in human monocytes is largest, and the expression frequencies of the remaining 99 genes are in the order of SEQ ID N0: 202 to 300.

Each of the gene groups provided by the above-mentioned inventions is each aggregate of the genes specified by a tag sequence of SEQ ID NO: 1 to NO:300 consecutive to the nucleotide sequence 5'-CATG-3' and the gene information comprising 14 bp is sufficient for identifying the full length of each gene and is useful, for example, for diagnosis, monitoring and judgement of prognosis of human infectious diseases and inflammatory diseases via cloning, protein expression and functional analysis thereof. In addition, the cDNA group and the oligonucleotide group are useful as the material for microarray, DNA chip, etc. for such a diagnosis, etc. Further, since the novel gene group has a high possibility of participating in the cause of human infectious diseases and inflammatory diseases, protein for which each of those gene codes and antibody and antagonist thereof are useful as the materials for development of pharmaceuticals.

### Brief Description of Drawing

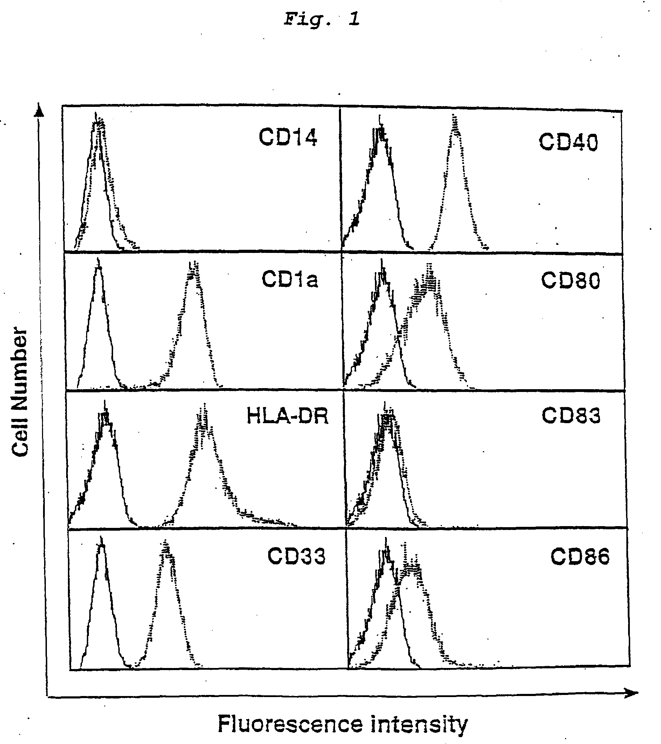

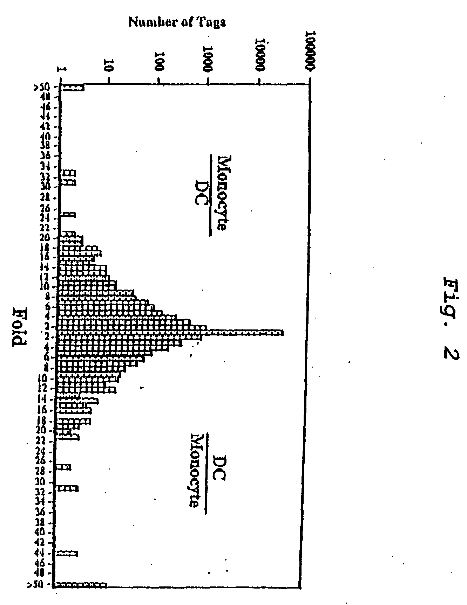

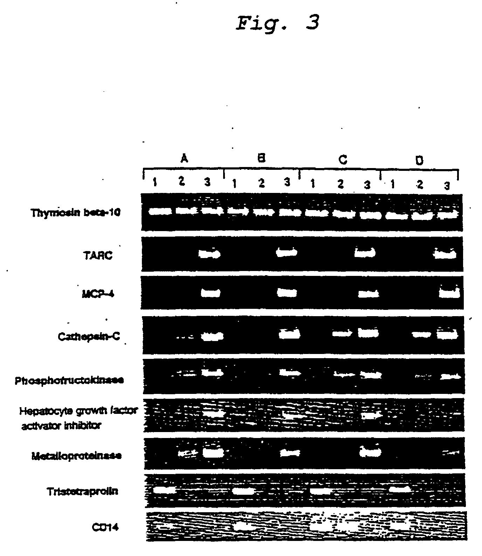

Fig. 1 is a result of a flow cytometry where the surface type of DCs derived from monocyte of human blood was analyzed; Fig. 2 is a result of comparison of gene expression in monocyte and DCs; and Fig. 3 is a result of the RT-PCR analysis of nine genes in monocyte, GM-macrophage and DCs of four blood donors (A, B, C and D).

### Best Mode for Carryig Out the Invention

Each of the gene groups expressed in the human dendritic cell according to this invention is an aggregate of 100 genes having different types expressed in the dendritic cell derived from human monocyte and has been identified by the known SAGE: serial analysis of gene expression method (Science 276:1268, 1997; Cell 88:243, 1997; Science 270:484, 1995; Nature 389:300, 1997; U. S. Patent 5,695,937). This SAGE method determines ten base pairs of DNA sequence (tag) which characterize each of cDNA prepared from genes expressing in any of the cells. It is also possible that, when the ratio of each tag is calculated from all of the resulting tag, expression frequency (copy numbers) of each gene is determined. Until now, the following gene expressions have been analyzed using this SAGE method. Thus, G2 arrest in colorectal cancer irradiation (Molecular Cell 1:3-11, 1997); oleate medium and pig 2q regulation (18th International Conference on Yeast Genetics and Molecular Biology s107:58, 1997); rat embryo fibroblast cells (Oncogene 15:1079-1085, 1997); p53 expression (Nature 389:300, 1997); yeast genome (Cell 88:243, 1997); and gastrointestinal tumors (Science 276:1268, 1997). The inventors of this application also specified the gene group which most abundantly expresses in human monocyte and human macrophage and the gene group which has a difference in terms of expression frequency between in human monocyte and in human macrophages by a SAGE method and have filed a patent application already (Patent Application No. 304550/1998).

Each of the gene groups expressed in human dendritic cell according to this invention is an aggregate of the genes specified by a method mentioned in the above-mentioned literatures and U. S. Patent, and is a gene aggregate where each cDNA contains the nucleotide sequences of SEQ ID NO:1-100, No:101-200 and NO:201-300, respectively, consecutive to 5'-CATG-3' closest to the poly A region. Those genes can be isolated, for example, by means of a screening of a human genomic library using each oligonucleotide comprising 14 bp of this invention as a probe.

Further, the cDNA group of this invention is a cDNA of the gene constituting each of the above-mentioned gene groups and is an aggregate of cDNA (or a partial sequence thereof) having SEQ ID NO:1-100, NO:101-200 and NO:201-300 each by connecting to 5'-CATG-3' closest to the poly A region. Such a cDNA can be isolated, for example, by means of a screening of a human cDNA library using each oligonucleotide comprising 14 bp of this invention as a probe.

The information of 14 bp constituting each of the oligonucleotides of this invention is sufficient for identifying the full length of each gene and cDNA.

Incidentally, the cDNA group of this invention includes a DNA fragment (10 bp or longer) containing any of the partial nucleotide sequences of each cDNA. In addition, DNA fragment comprising sense and antisense strands are included in this cDNA group as well.

Usually, polymorphism due to the difference among individuals is frequently noted in human gene. Accordingly, gene and cDNA where one or more nucleotide(s) is/are added, deleted and/or substituted with other nucleotide or modified nucleotide in the gene group and cDNA group of this invention are included within the coverage of this invention.

The novel gene group of this invention is an aggregate of genes which have no corresponding genes as a result of retrieval of database and that which are specified only by means of EST (expression sequence tag). Such novel gene group can be concretely specified, for example, by a positional cloning using each oligonucleotide of this invention as a marker, by a screening of a cDNA library using the oligonucleotide as a probe, by a retrieval of a gene bank, etc. It is also possible to prepare a novel protein for which a novel gene codes and an antibody to this novel protein from the novel gene. The protein can, for example, be prepared by a method where the novel gene cDNA or a partial sequence thereof is subjected to an *in vitro* transcription or a method where it is expressed in a large quantity by a suitable host-vector. The antibody can be also prepared as a polyclonal antibody or a monoclonal antibody according to a known method.

The antagonist of this invention is one to an expression of each gene belonging to each gene group of this invention and, for example, it is an antagonist to a transcription factor of each gene. Further, the antagonist of this invention is one to an activity of the protein expressed by each gene belonging to each gene group and is, for example, an antagonist to a receptor of each protein or an antagonist to a receptor of a target protein to which each protein in bonded. Thus, the gene belonging to each gene group of the invention is a gene which is abundantly expressed in human DCs or an aggregate of genes which is expressed abundantly or in a small amount as compared with in human monocyte and, since there is a high possibility that it is participated in onset or progress of human infectious diseases, inflammation, etc., the antagonist to those gene and protein is useful as an ingredient of the pharmaceuticals for prevention or therapy of infectious diseases and inflammation.

Incidentally, the antagonist can be specified, for example, by an *in* *vitro* expression system of each of the above-mentioned genes or by a known screening method where each protein is a subject. Alternatively, it can be prepared by means of chemical synthesis, etc. as a result of structural analysis, etc. of the protein or a receptor thereof.

As hereunder, concrete specifying methods for each gene group of this invention and the specified gene groups will be illustrated.

### (A) Preparation of cells

Peripheral blood mononuclear cells (PBMC) were extracted by a conventional method from venous blood of eight healthy persons and passed through a magnetic cell separation system to separate the monocyte and a suspension of the cells was incubated to give a highly purified monocyte.

Further, the monocyte was incubated together with GM-CSF according to a conventional method to prepare macrophage (GM-macrophage).

In addition, the monocyte which was positive to peripheral blood CD14 was incubated for five days together with GM-CSF, IL-4 and TNF-α whereupon DCs were prepared. Thus, under such an incubating condition, monocyte was differentiated to non-adhesive 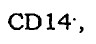 CD1a⁺, CD80^{low/l}, CD86^{low/-}, HLA-DR⁺, CD33⁺ and 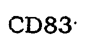 (cf. Fig. 1). Those cells had a dendritic form of immature DCs.

### (B) SAGE analysis

mRNA was prepared from each of the cells prepared in the above (A) and cDNA was synthesized using a biotinated oligo(dT). The cDNA (DC library) was digested by a restriction enzyme NIaIII and the 3'-terminal of each cDNA was purified using avdin-magnet beads. Since the cleaved site by NIaIII is 5'-CATG-3', the sequence at the 5'-terminal of the purified cDNA 3'-terminal piece is always CATG. After that, cDNA was divided into two, different linker was added to 5'-terinal of each of them and the cDNA was cleaved at the site which was 14 base pairs downstream from the 3'-terminal of the linker using a restriction enzyme BsmF1. All of those DNA fragments of 14 base pairs were common in that their 5'-terminal was a CATG sequence but, since the residual ten base pairs were different for each cDNA, the ten-base pair sequence becomes a tag for characterizing the cDNA. In order to clone the aggregate of those tags, the cleaved site of BsmF1 was made into a blunt terminal, cDNA fragments divided into two were ligated and DNA was amplified by means of a PCR using an oligonucleotide for each linker as a primer. As a result, there were manufactured DNA fragments (Ditag) wherein linker sequence (5'-GGATG-3'), 5'-CATG-3' and complementary sequences thereof were available at both terminals and different tag sequences were linked at each of the 3'-terminals. The amplified DNA fragments were cleaved by NIaIII, then each Ditag was ligated to prepare a concatemer and it was cloned to a vector pZero1.

### (C) Gene group expressed in human DCs

According to the above operation, 58,540 tags were obtained from cDNA expressed in human DCs, 17,000 or more genes were specified by those tags and, among them, there were 5,000 or more genes in which same tags were present twice or more. From all of those tags, 100 tag were selected in the order of abundance of duplications (i.e., high expression frequency of cDNA designated by the tag) and shown in SEQ ID NO:1 to NO: 100.

As will be apparent from the above description, the tags of SEQ ID NO: 1 to NO: 100 are ten base pairs which continuously exist in the nucleotide sequence CATG existing in the position nearest the poly A region of the gene cDNA expressing in human DCs. Accordingly, when the known DNA database is searched and cDNA having a know sequence is retrieved, the nucleotide sequence CATG which is nearest the poly A region of the DNA sequence is found and subjected to a matching with ten nucleotide sequences connecting thereto whereupon it is possible to specify the cDNA designated by the tag (and gene coding for the cDNA). Table 1 and Table 2 are tags (column B), numbers of the tag (column A) of SEQ ID NO:1 to NO: 100, gene (column C) specified as a result of searching the database depending upon each tag and registration numbers (columns D, E and F) of the database.

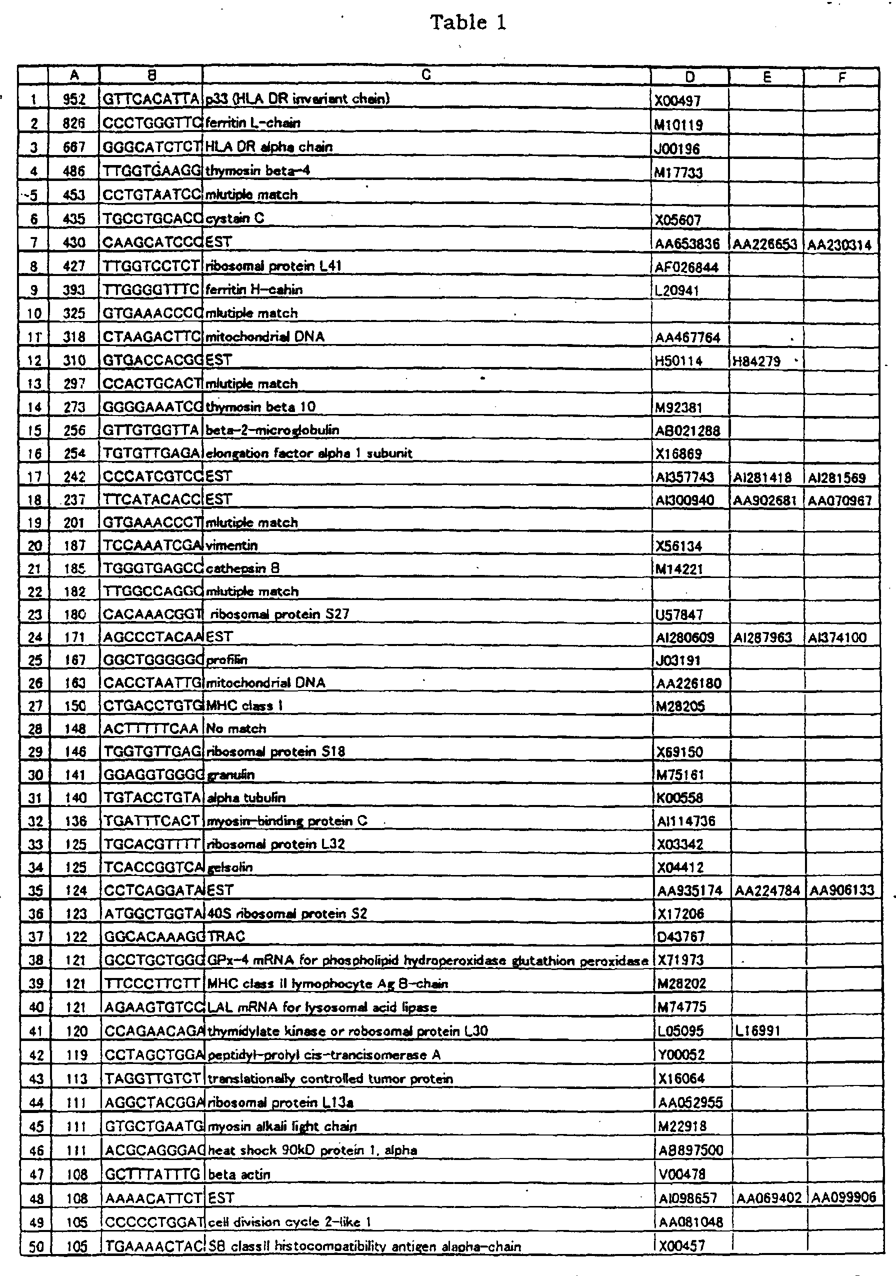

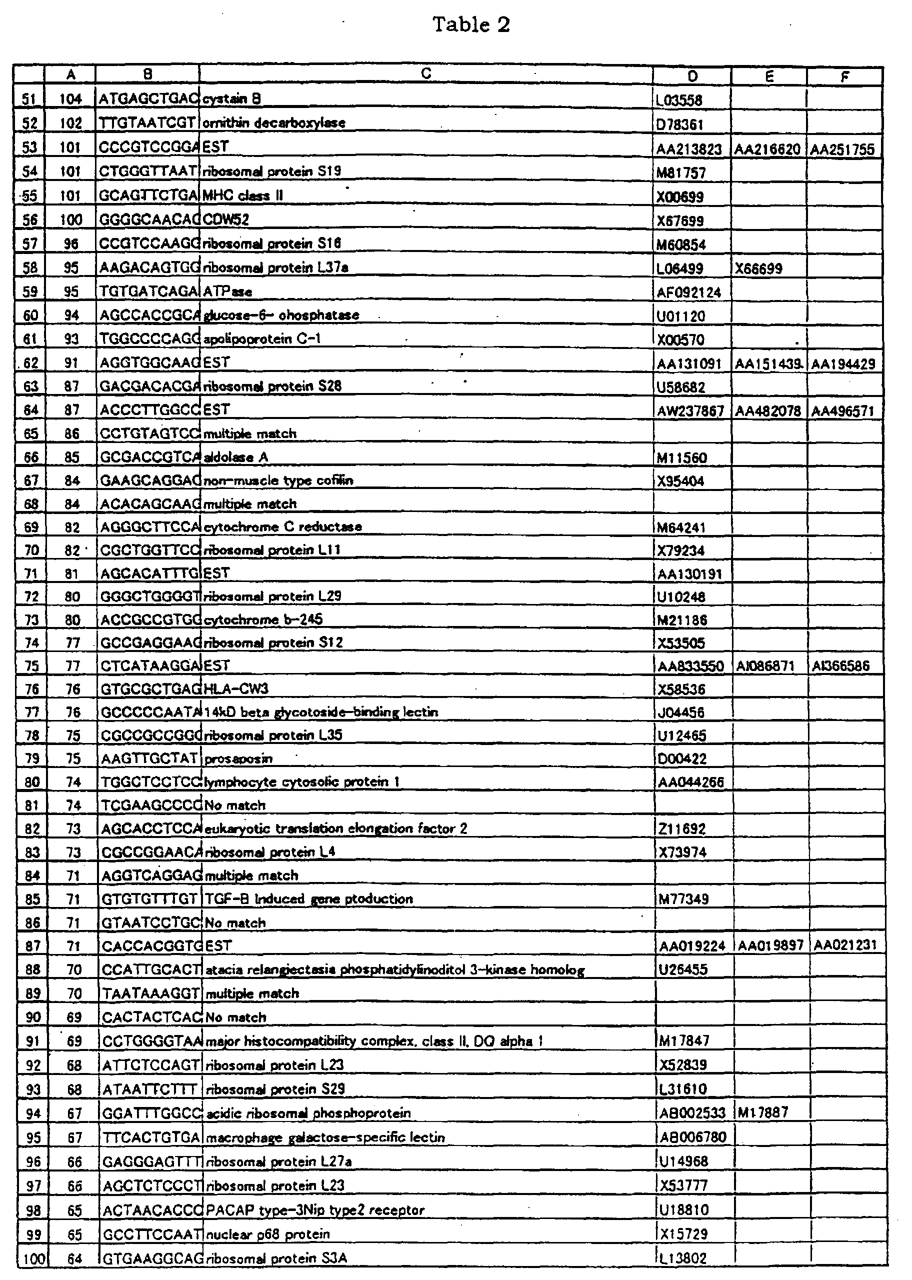

As shown in Table 1 and Table 2, the gene which was most abundantly expressed in human DCs was an HLA DR invariant chain (expression frequency: 1.17%). It was confirmed that the genes which were expressed on the whole in a DC library were related to MHC Class I, Class II, protein synthesis and cell skeleton.

### (D) Gene group abundantly expressed in human DCs as compared with in human monocyte

By the same manner as mentioned in the above (B), the tag for each genes expressing in human monocyte and human GM-macrophage were specified and the expressed genes in human monocyte and human DCs were compared. In those cells, most of the genes showed a similar expression pattern (cf. Fig. 2). However, with regard to 313 genes expressed in monocyte, the expression pattern in DCs was significantly (p <0.01) different. Thus, among the 131 genes, the expression level of 181 genes lowered in DCs. On the contrary, 132 genes were expressed in a higher frequency in DCs than in monocyte. Table 3 and Table 4 show the preceding 100 genes which were more abundantly expressed in human DCs than in human monocyte. In Tables 3 and 4, genes expressed in GM-macrophage and in DCs were shown and compared as well. Thus, in Tables 3 and 4, column A is an expression frequency of tags in monocyte and DCs in terms of "fold"; column B is copy numbers of tags in monocyte; column C is copy numbers of tags in GM-macrophage; column D is copy numbers of tags in DCs; column E is a tag sequence (SEQ ID NO:101 to NO:200); column F is a protein accorded in GenBank data; and columns G, H and I are registration numbers at the GenBank.

Incidentally, in Table 4, the full name of the protein in line 23, column F is "tyrosine 3-monooxygenase/tryptophan 5-monooygenase activation protein, beta polypeptide" while that in line 95, column F is "*Homo sapiens* clone 24860 Ena-VASP like protein mRNA sequence, partial cds".

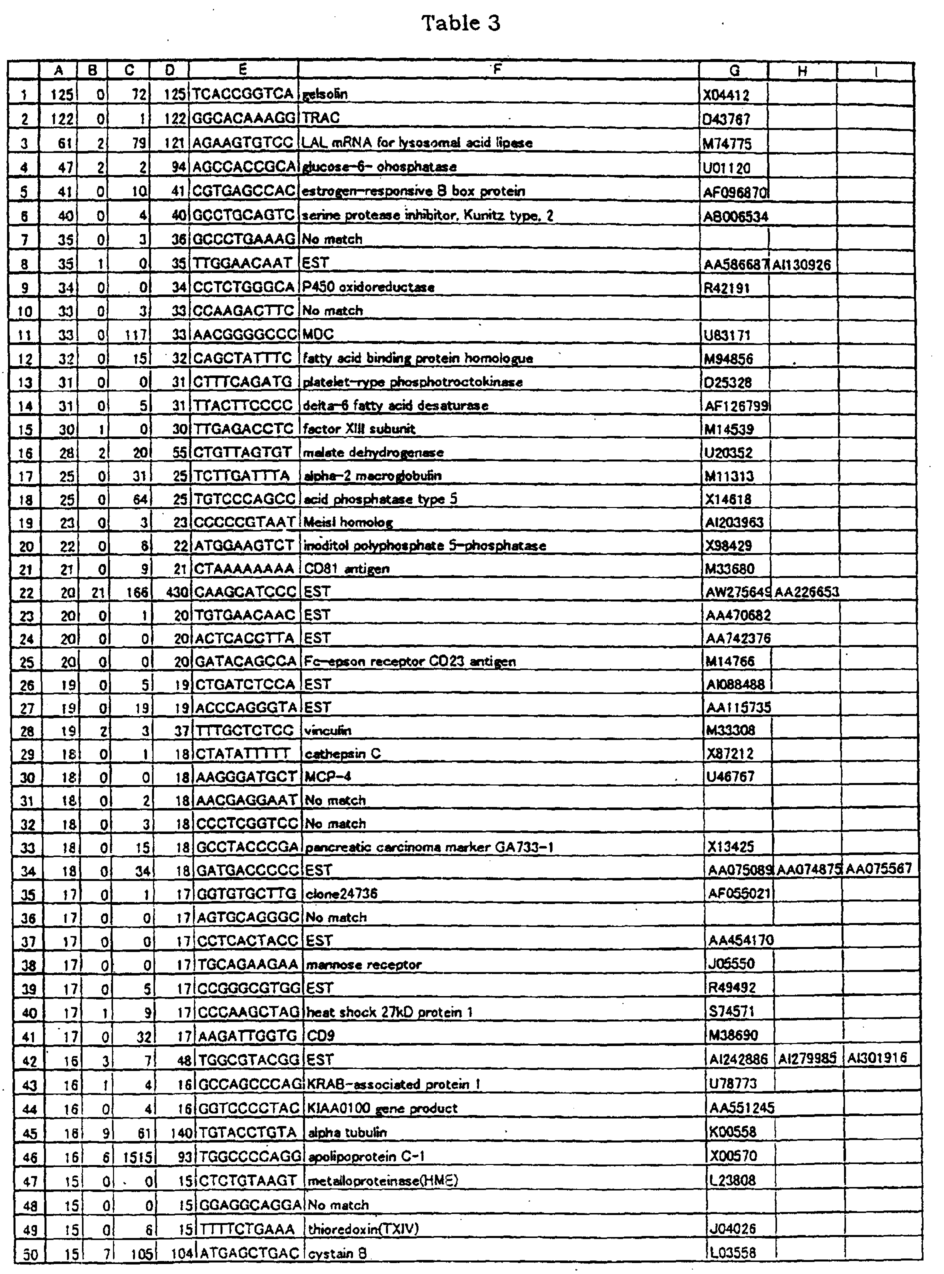

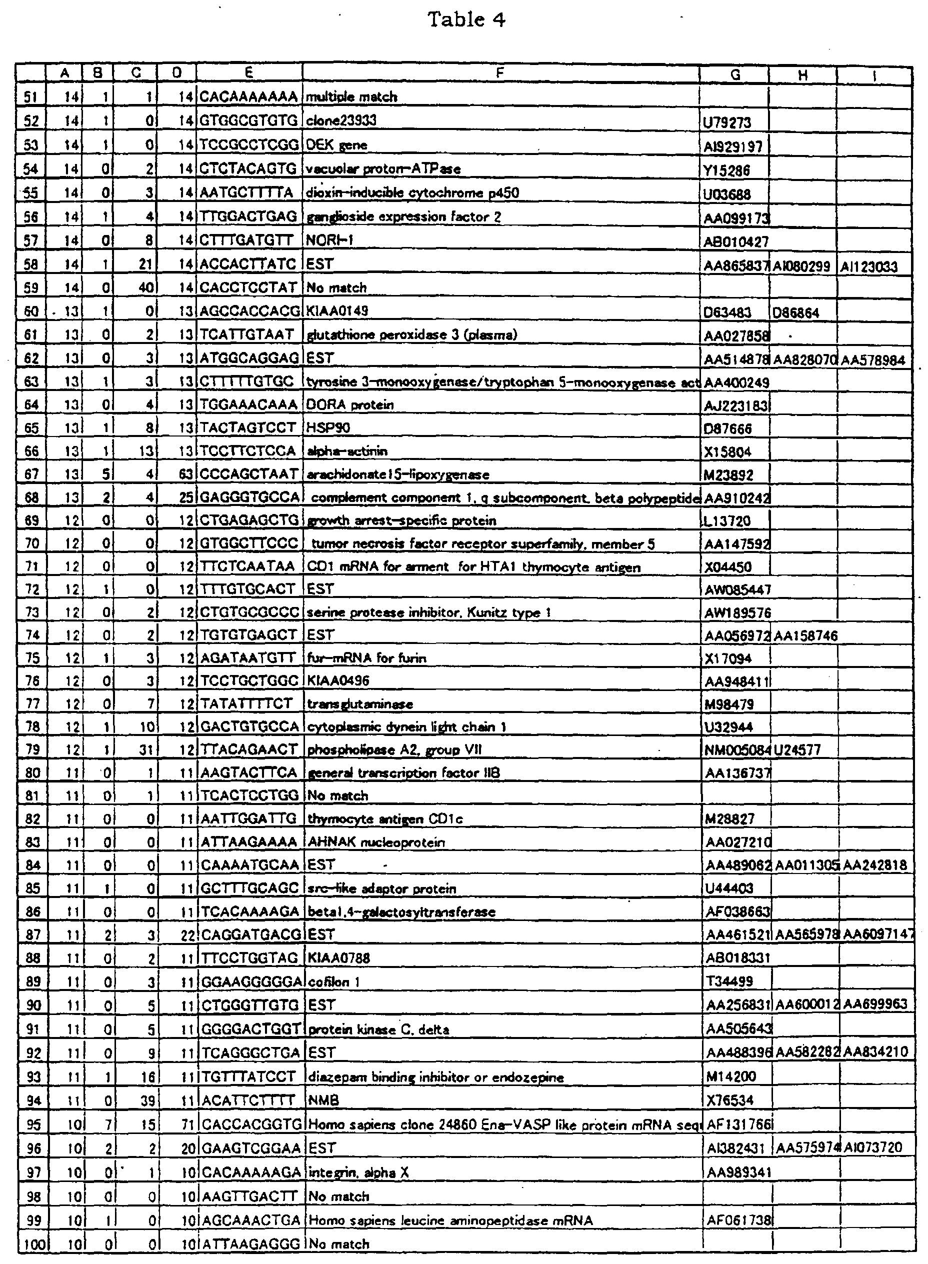

As shown in Table 3 and Table 4, GM-macrophage and DCs showed the similar gene expression patterns. On the other hand, the gene showing higher expression frequency in DCs as compared with in monocyte was a gene coding for the proteins participated in cell structures such as gelsolin and vinculin, the proteins participated in lipid metabolism such as lysosome acid lipase and apolipoprotein C-1 and the chemokines such as TARC and MDCMCR-4. In addition, gelsolin, lysosome acid lipase, MDC, fatty acid-bonded protein homologs, acidic phosphatase type 5, GA 733-1, CD 9, HPS 27, etc. increased both in macrophage and DCs. On the other hand, expression in a high frequency is specifically noted in TARC, hepatocyte growth accelerating inhibiting factor type 2 (HAI-2), phosphofructokinase of a platelet type, factor XIII, CD 23, cathepsin C, MCP-4 and metalloprotease (cf. Fig. 3).

### (E) Gene group which is less expressed in human DCs as compared with in human monocyte

Genes expressed in human monocyte and human DCs were compared by the same manner as mentioned in the above (D) and 100 genes where the expression frequency decreased in DCs as compared with in monocyte are shown in Tables 5 and 6. Form of indication is the same as that in Table 3 and 4. Incidentally, in Table 6, line 58 of column F is "nuclear factor of kappa light polypeptide gene enhancer in B-cells inhibitor, alpha" and line 59 of column F is "monocyte activation antigen or urokinase-type plasminogen activator receptor".

Genes where the expression was most reduced in DCs were complement proteins (ficolin and properdin), DNA-bonded proteins (GOS3, GOS2 and c-fos) and a surface protein (CD14). The genes where expression was reduced in DCs showed the same tendency in GM-macrophage as well. Further, the genes where a reduction of expression in 10-fold or more as compared with GM-macrophage was noted were MRP-14, MRP-1α, CD14, alpha 1 antitypsin, HLA-B and tranleolase. Incidentally, a reduction in CD14 in DCs was also confirmed by the result of a flow cytometry analysis as shown in Fig. 1.

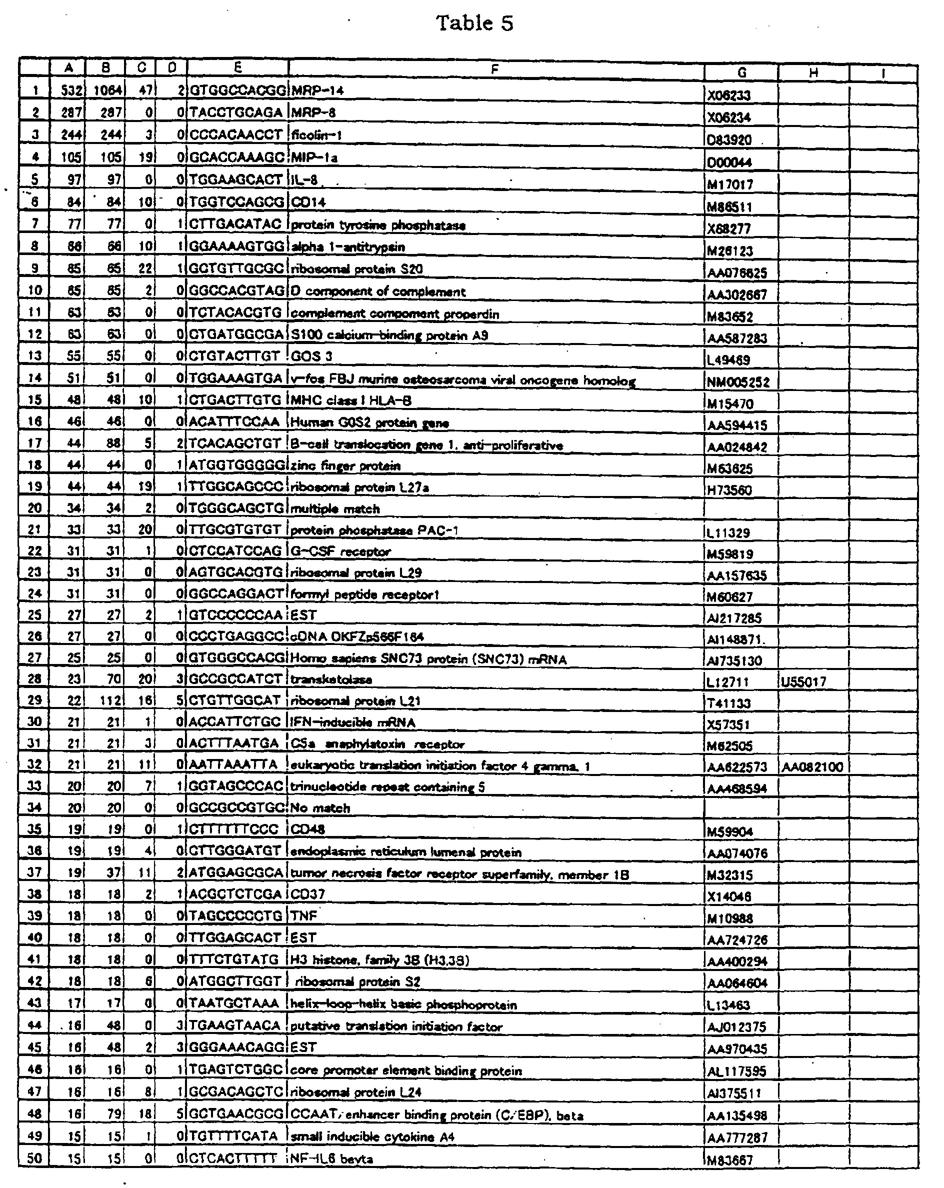

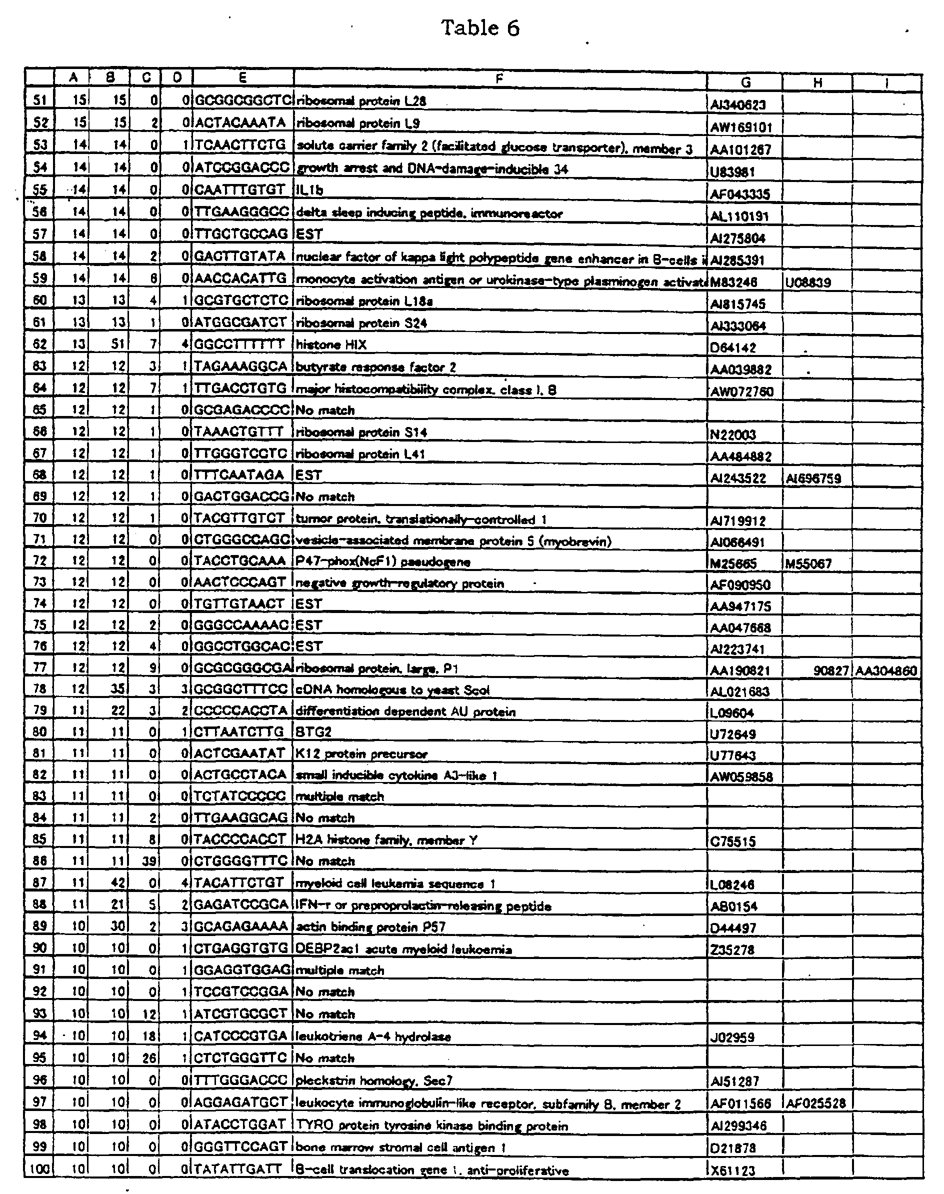

Incidentally, an SAGE analysis of each gene expression as mentioned above was carried out using the cells prepared from the blood of the eight persons. Now, in order to check whether the difference in each gene expression is dependent upon the difference in gene of blood donors, an RT-PCR was carried out for the blood samples for four persons with a target of 9 genes. The result is as shown in Fig. 3 and it was confirmed that the result of the SAGE analysis was not due to the difference in the gene of the blood donor but was dependent upon the difference in the gene expression frequencies in monocyte, GM-macrophage and DCs.

Further, the cDNA group of this application is an aggregate of cDNAs of gene constituting the above-mentioned each gene group connecting to 5'-CATG-3' which is nearest nucleotide sequence to the poly A region having the nucleotide sequence of SEQ ID NO:1 to NO:100, NO:101 to NO:200 and NO:201-NO:300 or a partial sequence thereof. The cDNA group of this invention covers DNA fragments (10 bp or more) containing any partial nucleotide sequence of each cDNA. Further, DNA fragment comprising sense chain and antisense chain is covered by this cDNA group as well.

In human genes, polymorphism due to the difference among individuals is frequently noted in general. Accordingly, in the gene group and cDNA group of this invention, gene and cDNA where one or more nucleotide(s) is/are added, deleted and/or substituted with other nucleotide are also included within a coverage of this invention.

As mentioned hereinabove, all of the gene groups provided by this invention including novel genes are genes which are expressed in human DCs and, particularly, the preceding 100 genes having the most abundant expressing amount. Further, the gene group provided by this invention is a gene group having a relatively high expressing amount and that having a relatively low expressing amount in human DCs (and GM-macrophage) as compared with in human monocyte. Accordingly, those gene group and cDNa group as well as the aggregation of tags designating them are useful, for example, in the following fields.

### (1) Specification of novel genes

With regard to the novel gene (group) provided by this invention, it is possible to specify such novel gene by means of, for example, a positional screening using an oligonucleotide where 5'-CATG-3' is added to the 5'-side of its tag sequence as a marker, a screening of a cDNA library using the said oligonucleotide as a marker or a search of the gene bank. It is also possible from the novel gene to prepare a novel protein for which the gene codes and also an antibody and an antagonist to the said protein. The protein can be prepared, for example, by a method where the novel gene cDNA or a partial sequence thereof is subjected to an *in vitro* transcription or by a method where expression in a large quantity is carried out using an appropriate host-vector. With regard to an antibody, it can be prepared as a polyclonal antibody or as a monoclonal antibody according to a known method. With regard to an antagonist, it can be prepared by means of chemical synthesis, etc. as a result of elucidation of structure of the protein or the receptor thereof.

### (2) Specification of morbid gene

There is a high possibility that the causative gene for the diseases in which human DCs are participated (such as atopic dermatitis and asthma) is a gene which is expressed in a high frequency in DCs. That is, for example, an excessive expression of the specific gene contained in the gene group expressed in DCs provided by this invention, decrease or deletion of expressed amount, gene mutation, etc. In addition, as mentioned already, there are genes showing clearly different amounts between in monocyte and in DCs. Accordingly, such genes showing different expressing amount are also the candidates for useful gene for the diseases in which each cell function is participated. As such, each gene group provided by this invention is useful for clarification of those morbid gene and an onset mechanism thereof.

### (3) Development of pharmaceuticals

Clarification of the causative gene and of the onset mechanism as mentioned in the above (2) is useful in the development of therapeutic agents for the above-mentioned diseases. Examples are pharmaceuticals which lower the expressing amount of excessively expressing gene.

### (4) Development of diagnostic means

There is a usefulness in the development of a diagnostic means for the diseases in which human DCs are participated. Examples are a kit for EIA and RIA utilizing an antibody to protein expressed by the causative gene and a DNA chip in which a partial or a full-length DNA of the gene is incorporated. Particularly, a DNA chip is useful for diagnosis of the diseases in which plural genes are participated but, since the DNA fragment numbers which can be incorporated on the substrate are limited, it is necessary to select the gene which is essential for the diagnosis. This invention provides gene and cDNA having an abundant expressing amount and, since it goes without saying that they are an aggregate of gene playing an important role in cell functions and cDNA thereof, they are quite useful as the candidates for DNA constituting a DNA chip. In addition, since the oligonucleotide group of this invention containing a tag sequence is a sequence which correctly specifies each gene, it can be also utilized as a minimum DNA sequence being incorporated in a DNA chip.

Further, the gene information comprising 14 bp of a novel gene provided by this invention is an information which is sufficient for identifying the full length of each gene and it relates to the development of pharmaceuticals and antagonists via cloning, protein expression and function analysis thereof.

### Industrial Applicability

In accordance with the invention of this application, there are provided a gene group comprising 100 genes which are highly expressed in human DCs and a gene group which is expressed in a high frequency and in a low frequency in human DCs as compared with in human monocyte. Those gene groups are quite useful not only for understanding of the differentiation process from monocyte to DCs but also for developing the novel means for diagnosis and therapy of various human diseases in which monocyte and DCs play an important role.

## Claims

1. A human dendritic cell-expressed gene group comprising 100 genes at the highest expression frequency among genes expressed in human dendritic cells derived from human monocytes, wherein the cDNAs of the individual genes individually contain nucleotide sequences of SEQ ID N0: 1 to 100 consecutive to the nucleotide sequence 5'-CATG-3' closest to the poly A region.

2. A human dendritic cell-expressed gene group of claim 1, wherein the expression frequency of the gene encoding the cDNA containing the nucleotide sequence of SEQ ID N0: 1 is the highest and the expression frequencies of the remaining 99 genes are in the order of SEQ ID N0: 2 to 100.

3. A human dendritic cell-expressed gene cDNA group comprising the cDNAs or partial sequences thereof of the individual genes composing a human dendritic cell-expressed gene group of claim 1, wherein the cDNAs individually contains nucleotide sequences of SEQ ID N0: 1 to 100 consecutive to the nucleotide sequence 5'-CATG-3' closest to the poly A region.

4. A human dendritic cell-expressed novel gene group included in a human dendritic cell-expressed gene group of claim 1, wherein the cDNAs of the individual novel genes individually contain nucleotide sequences of SEQ ID NO:7, 12, 17, 18, 24, 28, 35, 48, 53, 62, 64, 71, 75, 81, 86, 87 or 90 consecutive to the nucleotide sequence 5'-CATG-3' closest to the poly A region.

5. A novel protein encoded by the gene of the dendritic cell-expressed novel gene group of claim 4.

6. An antibody to the novel protein of claim 5.

7. An antagonist to the expression of each gene belonging to the gene group of claim 1.

8. An antagonist, to the activity of protein expressed by each gene belonging to the gene group of claim 1.

9. A group of oligonucleotides individually having the nucleotide sequence of SEQ ID NO:1 to NO:100 consecutive to the nucleotide sequence 5'-CATG-3'.

10. A human dendritid cell-expressed gene group comprising 100 genes expressed more in human dendritic cells derived from human monocytes than in human monocytes, wherein the cDNAs of the individual genes individually contain nucleotide sequence of SEQ ID NO:101 to 200 consecutive to the nucleotide sequence 5'-CATG-3' closest to the poly A region.

11. A human dendritic cell-expressed gene group of claim 10, wherein the difference between the expression frequency of the gene encoding the cDNA containing the nucleotide sequence of SEQ ID N0: 101 and the expression frequency of this gene in human monocytes is largest, and the expression frequencies of the remaining 99 genes are in the order of SEQ ID N0: 102 to 200.

12. A human dendritic cell-expressed gene cDNA group comprising the cDNAs or partial sequences thereof of the individual genes composing a human dendritic cell-expressed gene group of claim 10, wherein the cDNAs individually contains nucleotide sequences of SEQ ID N0: 101 to 200 consecutive to the nucleotide sequence 5'-CATG-3' closest to the poly A region.

13. A human dendritic cell-expressed novel gene group included in a human dendritic cell-expressed gene group of claim 10, wherein the cDNAs of the individual novel genes individually contain nucleotide sequences of SEQ ID NO: 107, 108, 110, 122-124, 126, 127, 131, 132, 134, 136, 137, 139, 142, 148, 158, 159, 162, 172, 174, 181, 184, 187, 190, 192, 196, 198 or 200 consecutive to the nucleotide sequence 5'-CATG-3' closest to the poly A region.

14. A novel protein encoded by the gene of the dendritic cell-expressed novel gene group of claim 13.

15. An antibody to the novel protein of claim 14.

16. An antagonist to the expression of each gene belonging to the gene group of claim 10.

17. An antagonist to the activity of protein expressed by each gene belonging to the gene group of claim 10.

18. A group of oligonucleotides individually having the nucleotide sequence of SEQ ID NO:101 to NO:200 consecutive to the nucleotide sequence 5'-CATG-3'.

19. A human dendritid cell-expressed gene group comprising 100 genes expressed less in human dendritic cells derived from human monocytes than in human monocytes, wherein the cDNAs of the individual genes individually contain nucleotide sequence of SEQ ID NO:201 to 300 consecutive to the nucleotide sequence 5'-CATG-3' closest to the poly A region.

20. A human dendritic cell-expressed gene group of claim 19, wherein the difference between the expression frequency of the gene encoding the cDNA containing the nucleotide sequence of SEQ ID N0: 201 and the expression frequency of this gene in human monocytes is largest, and the expression frequencies of the remaining 99 genes are in the order of SEQ ID N0: 202 to 300.

21. A human dendritic cell-expressed gene cDNA group comprising the cDNAs or partial sequences thereof of the individual genes composing a human dendritic cell-expressed gene group of claim 10, wherein the cDNAs individually contains nucleotide sequences of SEQ ID N0: 201 to 300 consecutive to the nucleotide sequence 5'-CATG-3' closest to the poly A region.

22. A human dendritic cell-expressed novel gene group included in a human dendritic cell-expressed gene group of claim 10, wherein the cDNAs of the individual novel genes individually contain nucleotide sequences of SEQ ID NO:225, 234, 240, 245, 257, 265, 268, 269, 274-276, 284, 286, 292, 293 or 295 consecutive to the nucleotide sequence 5'-CATG-3' closest to the poly A region.

23. A novel protein encoded by the gene of the dendritic cell-expressed novel gene group of claim 22.

24. An antibody to the novel protein of claim 23.

25. An antagonist to the expression of each gene belonging to the gene group of claim 19.

26. An antagonist to the activity of protein expressed by each gene belonging to the gene group of claim 19.

27. A group of oligonucleotides individually having the nucleotide sequence of SEQ ID NO:201 to NO:300 consecutive to the nucleotide sequence 5'-CATG-3'.
